# EUROPEAN PATENT APPLICATION

(11) **EP 2 767 235 A1**
(43) Date of publication of application: **20.08.2014**
(21) Application number: 12839788.2
(22) Date of filing: 26.09.2012
(51) Int. Cl.: A61B 5/16, A61B 5/0402, A61B 5/0408, A61B 5/0476, A61B 5/0478, A61B 5/0488, A61B 5/087, A61B 5/11, A61B 5/1455

(54) **PSG-TEST HEADGEAR AND PSG TEST DEVICE**

(30) Priority: 12.10.2011 JP 2011224792
(71) Applicant: Sony Corporation, Tokyo 108-0075 (JP)
(72) Inventor: WADA, Seiji, Tokyo 108-0075 (JP); YAMAMOTO, Takuro, Tokyo 108-0075 (JP); SOMA, Haruhiko, Tokyo 108-0075 (JP); NAKASHIMA, Yusaku, Tokyo 108-0075 (JP); TOMITA, Takashi, Tokyo 108-0075 (JP); NAKAMURA, Mitsuhiro, Tokyo 108-0075 (JP); KIMURA, Natsuki, Tokyo 108-0075 (JP)
(74) Representative: DeVile, Jonathan Mark
(86) International application number: PCT/JP2012/006128
(87) International publication number: WO 2013/054480

(57) **Abstract**

[Object]

To provide a PSG test apparatus suitable for home-monitoring.

[Solving means]

A PSG test headgear according to the present disclosure includes an electroencephalogram electrode, an electrooculogram electrode, and an oxygen saturation sensor. The electroencephalogram electrode acquires an electroencephalogram of a user. The electrooculogram electrode acquires an electrooculogram of the user. The oxygen saturation sensor acquires an oxygen saturation of the user.

## Description

### [Technical Field]

The present disclosure relates to a PSG test headgear and a PSG test apparatus for obtaining an all-night polysomnogram used for judging a sleep state of a user.

### [Background Art]

A PSG (Polysomnography) test is a test for evaluating an overall sleep state and is used for judging a sleep apnea syndrome. Specifically, the PSG test involves measuring a plurality of measurement items including an electroencephalogram, an electrooculogram, a chin electromyogram, an oxygen saturation, naso-oral breathing, and thoraco-abdominal breathing at the same time.

Which of the measurement items is to be measured is defined as in Type 1 (Standard Full PSG), Type 2 (Comprehensive portable PSG), and the like according to a measurement environment by American Academy of Sleep Medicine (AASM), for example.

Since there are a wide variety of measurement items for the PSG test as described above, it is assumed that the measurements are carried out by engineers in specially-equipped facilities such as a hospital. In Patent Literature 1 or Patent Literature 2 below, there is disclosed a measurement apparatus for measuring several measurement items for the PSG test.

### [Citation List]

### [Patent Literature]

[PTL 1]
   Japanese Patent Application Laid-open No. 2004-305258
[PTL 2]
   Japanese Patent Application Laid-open No. 2008-067892

### [Summary]

### [Technical Problem]

However, a measurement apparatus that enables the PSG test to be performed not in a specially-equipped facility but at home by a user him/herself (home-monitoring) is being desired. In a measurement apparatus as that disclosed in Patent Literature 1 or Patent Literature 2, the test apparatus is of a large scale or the measurement items are insufficient, and thus measurements in a specially-equipped facility are assumed.

In view of the circumstances as described above, it is an object of the present disclosure to provide a PSG test apparatus suitable for home-monitoring.

### [Solution to Problem]

To attain the object described above, according to an embodiment of the present disclosure, there is provided a PSG test headgear including an electroencephalogram electrode, an electrooculogram electrode, and an oxygen saturation sensor.
The electroencephalogram electrode acquires an electroencephalogram of a user. The electrooculogram electrode acquires an electrooculogram of the user.
The oxygen saturation sensor acquires an oxygen saturation of the user.

With this structure, by the user wearing the PSG test headgear, an electroencephalogram, an electrooculogram, and an oxygen saturation can be acquired, and thus it becomes possible to acquire an all-night polysomnogram (PSG) in combination with measurement data from other measurement apparatuses.

The PSG test headgear may further include a headband that the user wears on a head, the electroencephalogram electrode may be provided at a predetermined position on the head of the user by the headband, the electrooculogram electrode may be provided at a temple of the user by the headband, and the oxygen saturation sensor may be provided at a forehead of the user by the headband.

With this structure, by the user wearing the PSG test headgear, the electroencephalogram electrode, the electrooculogram electrode, and the oxygen saturation sensor can be provided at appropriate positions.

The electroencephalogram electrode may be provided at electrode positions of F₃, F₄, C₃, C₄, O₁, and O₂ defined by the International 10-20 system.

With this structure, by the user wearing the PSG test headgear, the electroencephalogram electrode can be provided at a PSG test electrode position recommended by American Academy of Sleep Medicine (AASM).

The PSG test headgear may further include a signal processing unit that generates an all-night polysomnogram from an acquired signal.

With this structure, an all-night polysomnogram can be generated in the PSG test headgear, and thus a different information processing apparatus for generating an all-night polysomnogram becomes unnecessary.

To attain the object described above, according to an embodiment of the present disclosure, there is provided a PSG test apparatus including a PSG test headgear, a PSG test chin-gear, and a PSG test chest-gear.
The PSG test headgear includes an electroencephalogram electrode that acquires an electroencephalogram of a user, an electrooculogram electrode that acquires an electrooculogram of the user, and an oxygen saturation sensor that acquires an oxygen saturation of the user.
The PSG test chin-gear includes a chin electromyogram electrode that acquires a chin electromyogram of the user and an air current sensor that acquires naso-oral breathing of the user.
The PSG test chest-gear includes an electrocardiogram sensor that acquires an electrocardiogram of the user and a breathing exercise sensor that acquires a breathing exercise of the user.

With this structure, by the user wearing the PSG test apparatus, measurement values requisite for creating an all-night polysomnogram can be acquired.

The PSG test headgear may further include a signal processing unit that generates an all-night polysomnogram from an acquired signal, and the PSG test chest-gear may further include a chest transmission unit that wirelessly transmits, to the signal processing unit, output signals of the electrocardiogram sensor and the breathing exercise sensor.

With this structure, an all-night polysomnogram can be generated in the PSG test headgear, and thus a different information processing apparatus for generating an all-night polysomnogram becomes unnecessary.

The PSG test headgear may further include an external transmission unit that wirelessly transmits, to an external apparatus, the all-night polysomnogram generated by the signal processing unit.

With this structure, the PSG test chest-gear and the PSG test headgear can be connected without hampering a sleep of the user.

### [Effects of Invention]

As described above, according to the present disclosure, a PSG test apparatus suitable for home-monitoring can be provided.

### [Brief Description of Drawings]

[Fig. 1]
   A schematic diagram showing a structure of a PSG test apparatus according to an embodiment of the present disclosure.
[Fig. 2]
   A schematic diagram showing an arrangement of an electroencephalogram electrode of the PSG test apparatus.
[Fig. 3]
   A block diagram showing a functional structure of the PSG test apparatus.
[Fig. 4]
   A schematic diagram showing an operation of the PSG test apparatus.
[Fig. 5]
   A schematic diagram showing data formats used in the PSG test apparatus.

### [Description of Embodiments]

A PSG test apparatus according to an embodiment of the present disclosure will be described.

### [Structure of PSG test apparatus]

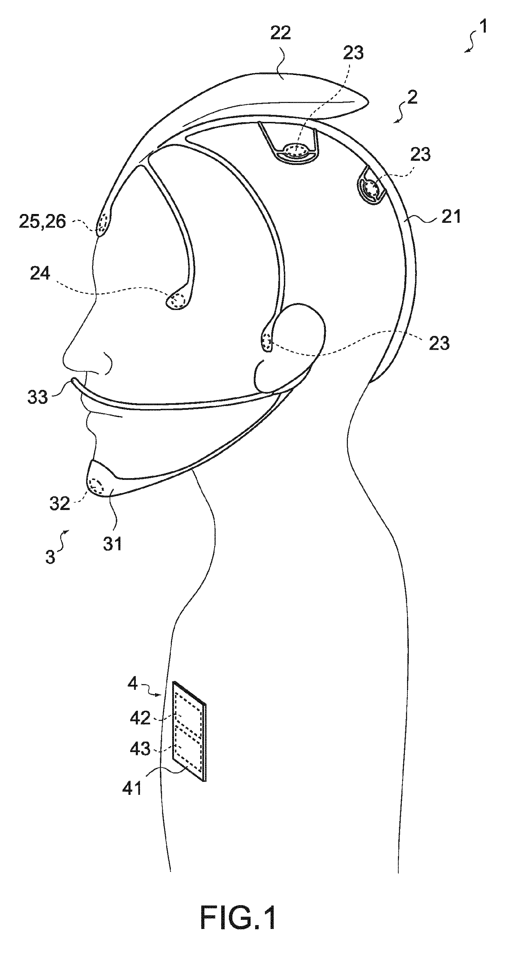

Fig. 1 is a schematic diagram showing a structure of a PSG test apparatus 1. As shown in the figure, the PSG test apparatus 1 includes a headgear 2, a chin-gear 3, and a chest-gear 4. The headgear 2 is worn by a user on a head, and the chin-gear 3 is worn by the user at a chin (jaw portion). The chest-gear 4 is worn by the user at a chest. The chin-gear 3 and the chest-gear 4 are connected to the headgear 2 in a wired or wireless manner.

As shown in Fig. 1, the headgear 2 includes a headband 21, a casing 22, an electroencephalogram electrode 23, an electrooculogram electrode 24, an oxygen saturation sensor 25, and a system reference electrode 26. The headband 21 can be worn by the user on the head, and the casing 22, the electroencephalogram electrode 23, the electrooculogram electrode 24, the oxygen saturation sensor 25, and the system reference electrode 26 are supported by the headband 21.

The headband 21 may be a member having a shape formed along the head of the user, for example, a plate-like shape curved from the forehead to the back of the head of the user and may be worn by the user on the head using its elasticity. Alternatively, shapes other than that described above may be used for the headband 21. It is favorable to use a headband 21 that does not deviate due to the user moving while sleeping.

The casing 22 is fixed to the headband 21 and incorporates therein a signal processing circuit and the like to be described later. Although the shape of the casing 22 is not particularly limited, a shape that does not hamper a sleep of the user is favorable.

The electroencephalogram electrode 23 is an electrode for acquiring an electroencephalogram (EEG) of the user. A plurality of electroencephalogram electrodes 23 may be provided in correspondence with measurement positions of the electroencephalogram. The electroencephalogram electrode 23 may be formed of an elastic member impregnated with an electrolyte solution and may also include a structure electrically connectable to the head surface of the user, for example. The electroencephalogram electrode 23 can be provided at a predetermined position by an arm extending from the headband 21.

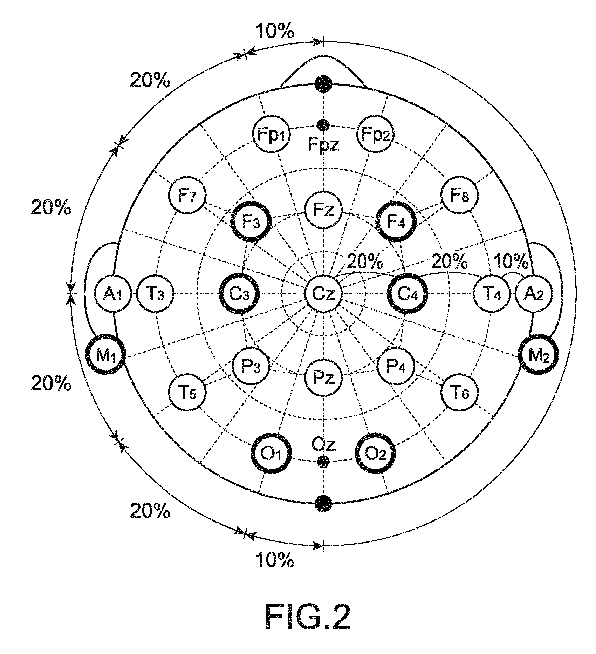

The electrode arrangement of the electroencephalogram electrode 23 is not particularly limited, but the following arrangement is favorable. Fig. 2 is a schematic diagram showing the electrode arrangement of the electroencephalogram electrode 23. In Fig. 2, an electrode arrangement defined by the International 10-20 system, which is a standard electrode arrangement for an electroencephalogram measurement, is shown. In the figure, F₃, F₄, C₃, C₄, O₁, and O₂ surrounded by bold lines show a recommended electrode arrangement for a PSG test recommended by American Academy of Sleep Medicine (AASM). Therefore, it is favorable for the electroencephalogram electrodes 23 to be arranged at such recommended positions. Moreover, reference electrodes (M₁, M₂) can be provided at earlobes of the user.

The electrooculogram electrode 24 is an electrode for acquiring an electrooculogram (EOG) of the user. The electrooculogram electrode 24 may be formed of an elastic member impregnated with an electrolyte solution and may be located at a temple of the user, for example. The electrooculogram electrode 24 can be provided at the temple of the user by the arm extending from the headband 21.

The oxygen saturation sensor 25 is a sensor for acquiring a percutaneous oxygen saturation (SpO₂). The oxygen saturation sensor 25 includes a light-emitting unit that irradiates light onto a skin and a light-receiving unit that measures reflected light, and a structure that judges an oxygen saturation from an absorbance difference of reflected light based on an oxygen concentration can be used. The oxygen saturation sensor 25 can be located at the forehead of the user by the arm extending from the headband 21.

The system reference electrode 26 is an electrode for acquiring a reference potential that is to be a reference for the electroencephalogram electrode 23, the electrooculogram electrode 24, and a chin electromyogram electrode 32 to be described later. The system reference electrode 26 may be formed of an elastic member impregnated with an electrolyte solution and can be located at the forehead or the like of the user, for example.

The headgear 2 has the structure as described above. It should be noted that a transceiver unit or signal processing unit to be accommodated in the casing 22 will be described later.

The chin-gear 3includes a support body 31, the chin electromyogram electrode 32, and a current sensor 33. The support body 31 can be worn by the user at the chin, and the chin electromyogram electrode 32 and the current sensor 33 are supported by the support body 31. The chin-gear 3 can be connected to the headgear 2 by a wiring.

The support body 31 may be a member having a shape formed along a face line of the user, for example, a shape formed from a lower earlobe portion of the user to a lower earlobe portion on the other side via the chin and can be worn by the user at the chin using its elasticity. Alternatively, shapes other than that described above may be used for the support body 31. It is favorable to use a support body 31 that does not deviate due to the user moving while sleeping.

The chin electromyogram electrode 32 is an electrode for acquiring a chin electromyogram (EMG). The chin electromyogram electrode 32 may be formed of an elastic member impregnated with an electrolyte solution, for example. The chin electromyogram electrode 32 can be incorporated into the support body 31 and provided at the chin of the user.

The current sensor 33 is a sensor for acquiring a naso-oral breathing flow of the user. The current sensor 33 may be a sensor that measures a differential pressure by an expiration. The current sensor 33 can be located by an arm extending between the nose and mouth of the user from the support body 31.

The chin-gear 3 has the structure as described above.

The chest-gear 4 includes a support body 41, an electrocardiogram sensor 42, and a breathing exercise sensor 43. The support body 41 can be worn by the user at the chest, and the electrocardiogram sensor 42 and the breathing exercise sensor 43 are supported by the support body 41. The chest-gear 4 can be connected to the headgear 2 in a wired or wireless manner.

The support body 41 has a shape with which the user can wear it at the chest and is fixed to the chest of the user by a belt wound around a body of the user, for example. Alternatively, shapes other than that described above may be used for the support body 41. It is favorable to use a support body 41 that does not deviate due to the user moving while sleeping.

The electrocardiogram sensor 42 is a sensor for acquiring an electrocardiogram (ECG) of the user. The electrocardiogram sensor 42 may be formed of an elastic member impregnated with an electrolyte solution and may also include a structure electrically connectable to the chest of the user, for example. The electrocardiogram sensor 42 may be incorporated into the support body 41 and provided at the chest of the user.

The breathing exercise sensor 43 is a sensor for acquiring a breathing exercise of the user. As the breathing exercise sensor 43, a sensor that measures an expansion and contraction of the belt used for fixing the support body 41 to the chest of the user, a sensor that measures an acceleration of the support body 41, or the like can be used arbitrarily.

The chest-gear 4 has the structure as described above.

### [Functional structure and operation of PSG test apparatus]

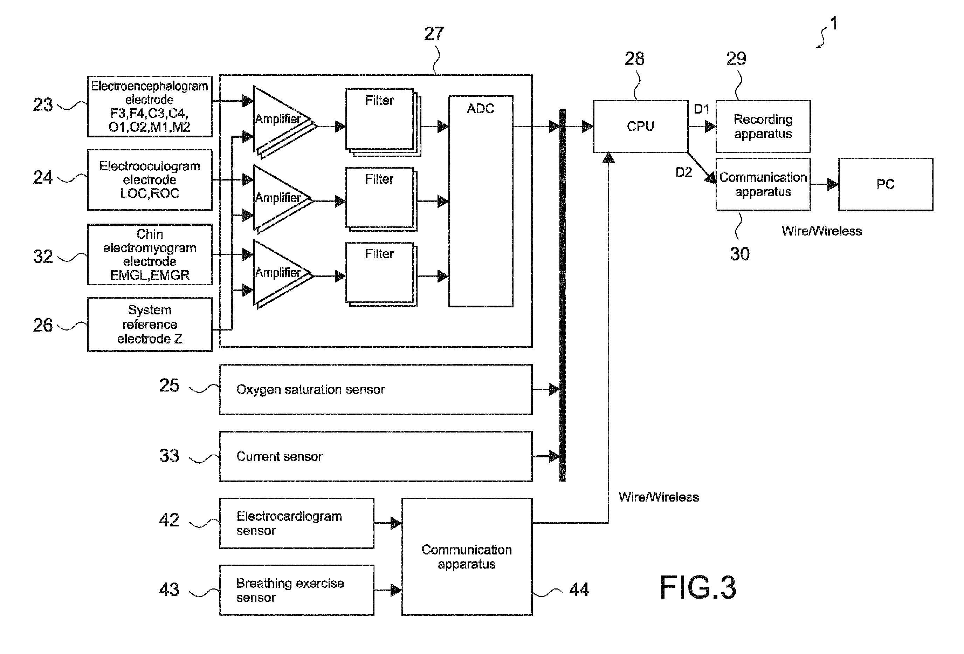

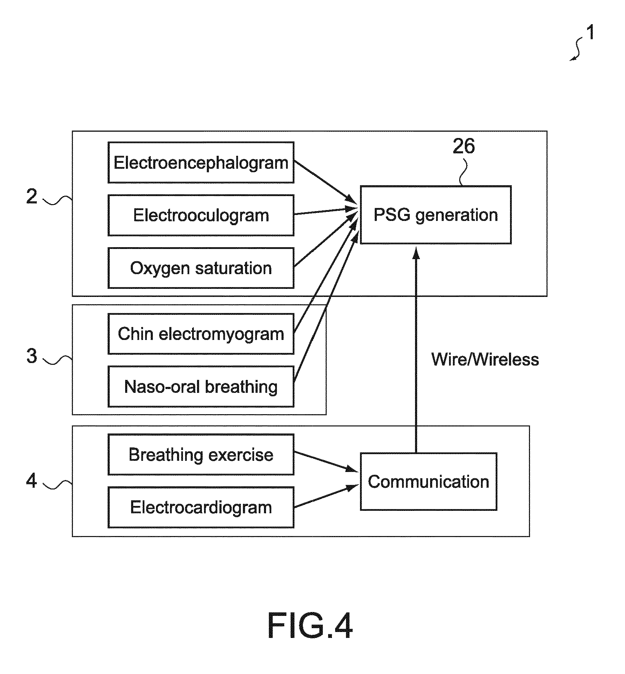

The functional structure of the PSG test apparatus 1 will be described. Fig. 3 is a schematic diagram showing a functional structure of the PSG test apparatus 1. Fig. 4 is a schematic diagram showing an operation of the PSG test apparatus 1.

As shown in Fig. 3, accommodated in the casing 22 of the headgear 2 are a signal processing circuit 27, a CPU (Central Processing Unit) 28, a recording apparatus 29, and an external communication apparatus 30. The signal processing circuit 27 is connected to the CPU 28, and the CPU 28 is connected to the recording apparatus 29 and the external communication apparatus 30.

The signal processing circuit 27 processes output signals of the electroencephalogram electrode 23, the electrooculogram electrode 24, and the chin electromyogram electrode 32. The signal processing circuit 27 includes amplifiers 271 connected to the electrodes, filters 272 connected to the amplifiers 271, and an ADC (Analog Digital Converter) 273 connected to the filters 272.

Outputs of the electroencephalogram electrode 23, the electrooculogram electrode 24, and the chin electromyogram electrode 32 are respectively input to the corresponding amplifiers 271 and subjected to a differential amplification with respect to the output of the system reference electrode 26. The outputs of the amplifiers 271 are input to the ADC 273 via the filters 272 and converted into digital signals. The generated digital signals are output to the CPU 28. Outputs of the oxygen saturation sensor 25 and the current sensor 33 are also input to the CPU 28.

Outputs of the electrocardiogram sensor 42 and the breathing exercise sensor 43 are transmitted to the CPU 28 by a chest communication apparatus 44 incorporated into the support body 41 of the chest-gear 4. The CPU 28 performs an aggregation and formatting of the output signals of the electrodes and sensors input as described above. The CPU 28 creates a recording format D1 and a communication format D2 as follows.

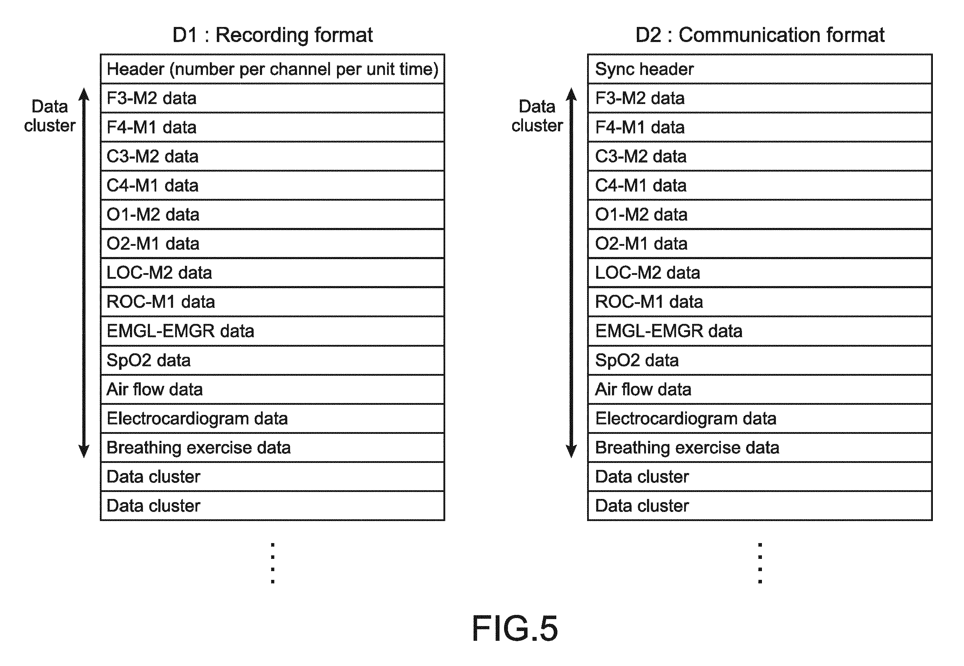

Fig. 5 shows an example of the recording format D1 and the communication format D2. The recording format D1 is constituted of a header that defines a data attribute and a cluster size in a data unit and a data body. The communication format D2 is constituted of a header indicating a head of a data cluster and a data body.

The CPU 28 outputs the recording format D1 to the recording apparatus 29 to be recorded and outputs the communication format D2 to the external communication apparatus 30 so that it is transmitted to an external apparatus (PC etc.). As a result, recording or real-time viewing of an all-night polysomnogram waveform becomes possible. In other words, with only the PSG test apparatus 1, PSG tests of Type 1 (Standard Full PSG) and Type 2 (Comprehensive portable PSG)defined by American Academy of Sleep Medicine (AASM) become possible.

The present disclosure is not limited to the embodiment described above and can be variously modified without departing from the gist of the present disclosure.

It should be noted that the present disclosure can also take the following structures.

(1) A PSG test headgear, including:
   an electroencephalogram electrode that acquires an electroencephalogram of a user;
   an electrooculogram electrode that acquires an electrooculogram of the user; and
   an oxygen saturation sensor that acquires an oxygen saturation of the user.
(2) The PSG test headgear according to (1) above, further including
   a headband that the user wears on a head,
   in which the electroencephalogram electrode is provided at a predetermined position on the head of the user by the headband,
   in which the electrooculogram electrode is provided at a temple of the user by the headband, and
   in which the oxygen saturation sensor is provided at a forehead of the user by the headband.
(3) The PSG test headgear according to (1) or (2) above,
   in which the electroencephalogram electrode is provided at electrode positions of F₃, F₄, C₃, C₄, O₁, and O₂ defined by the International 10-20 system.
(4) The PSG test headgear according to any one of (1) to (3), further including
   a signal processing unit that generates an all-night polysomnogram from an acquired signal.
(5) A PSG test apparatus, including:
   a PSG test headgear including an electroencephalogram electrode that acquires an electroencephalogram of a user, an electrooculogram electrode that acquires an electrooculogram of the user, and an oxygen saturation sensor that acquires an oxygen saturation of the user;
   a PSG test chin-gear including a chin electromyogram electrode that acquires a chin electromyogram of the user and an air current sensor that acquires naso-oral breathing of the user; and
   a PSG test chest-gear including an electrocardiogram sensor that acquires an electrocardiogram of the user and a breathing exercise sensor that acquires a breathing exercise of the user.
(6) The PSG test apparatus according to (5) above,
   in which the PSG test headgear further includes a signal processing unit that generates an all-night polysomnogram from an acquired signal, and
   in which the PSG test chest-gear further includes a chest transmission unit that wirelessly transmits, to the signal processing unit, output signals of the electrocardiogram sensor and the breathing exercise sensor.
(7) The PSG test apparatus according to (5) or (6) above,
   in which the PSG test headgear further includes an external transmission unit that wirelessly transmits, to an external apparatus, the all-night polysomnogram generated by the signal processing unit.

### [Reference Signs List]

- 1: PSG test apparatus
- 2: headgear
- 3: chin-gear
- 4: chest-gear
- 21: headband
- 22: casing
- 23: electroencephalogram electrode
- 24: electrooculogram electrode
- 25: oxygen saturation sensor
- 27: signal processing circuit
- 28: CPU
- 29: recording apparatus
- 30: external communication apparatus
- 31: support body
- 32: chin electromyogram electrode
- 33: current sensor
- 41: support body
- 42: electrocardiogram sensor
- 43: breathing exercise sensor
- 44: chest communication apparatus

## Claims

1. A PSG test headgear, comprising:
an electroencephalogram electrode that acquires an electroencephalogram of a user;
an electrooculogram electrode that acquires an electrooculogram of the user; and
an oxygen saturation sensor that acquires an oxygen saturation of the user.

2. The PSG test headgear according to claim 1, further comprising
a headband that the user wears on a head,
wherein the electroencephalogram electrode is provided at a predetermined position on the head of the user by the headband,
wherein the electrooculogram electrode is provided at a temple of the user by the headband, and
wherein the oxygen saturation sensor is provided at a forehead of the user by the headband.

3. The PSG test headgear according to claim 1,
wherein the electroencephalogram electrode is provided at electrode positions of F₃, F₄, C₃, C₄, O₁, and O₂ defined by the International 10-20 system.

4. The PSG test headgear according to claim 1, further comprising
a signal processing unit that generates an all-night polysomnogram from an acquired signal.

5. A PSG test apparatus, comprising:
a PSG test headgear including an electroencephalogram electrode that acquires an electroencephalogram of a user, an electrooculogram electrode that acquires an electrooculogram of the user, and an oxygen saturation sensor that acquires an oxygen saturation of the user;
a PSG test chin-gear including a chin electromyogram electrode that acquires a chin electromyogram of the user and an air current sensor that acquires naso-oral breathing of the user; and
a PSG test chest-gear including an electrocardiogram sensor that acquires an electrocardiogram of the user and a breathing exercise sensor that acquires a breathing exercise of the user.

6. The PSG test apparatus according to claim 5,
wherein the PSG test headgear further includes a signal processing unit that generates an all-night polysomnogram from an acquired signal, and
wherein the PSG test chest-gear further includes a chest transmission unit that wirelessly transmits, to the signal processing unit, output signals of the electrocardiogram sensor and the breathing exercise sensor.

7. The PSG test apparatus according to claim 6,
wherein the PSG test headgear further includes an external transmission unit that wirelessly transmits, to an external apparatus, the all-night polysomnogram generated by the signal processing unit.
